(19) Europäisches Patentamt
European Patent Office
Office européen des brevets

(11) **EP 1 121 145 B1**

(12) ## EUROPEAN PATENT SPECIFICATION

(45) Date of publication and mention
of the grant of the patent:
**17.04.2002 Bulletin 2002/16**

(21) Application number: **99948726.7**

(22) Date of filing: **15.10.1999**

(51) Int Cl.⁷: $A61K\ 38/28$

(86) International application number:
**PCT/DK99/00557**

(87) International publication number:
**WO 00/23099 (27.04.2000 Gazette 2000/17)**

(54) **INSULIN PREPARATIONS FOR PULMONARY DELIVERY CONTAINING MENTHOL**

INSULIN PRÄPARATIONEN ENTHALTEND METHANOL ZUR PULMONAREN VERABREICHUNG

PREPARATIONS D'INSULINE CONTENANT DU MENTHOL ET DESTINEES A L'ADMINISTRATION PAR VOIE PULMONAIRE

(84) Designated Contracting States:
**AT BE CH CY DE DK ES FI FR GB GR IE IT LI LU MC NL PT SE**
Designated Extension States:
**LT MK RO SI**

(30) Priority: **16.10.1998 DK 132698**

(43) Date of publication of application:
**08.08.2001 Bulletin 2001/32**

(73) Proprietor: **Novo Nordisk A/S**
**2880 Bagsvaerd (DK)**

(72) Inventor: **HAVELUND, Svend**
**DK-2880 Bagsvaerd (DK)**

(74) Representative: **Kellberg, Lars**
**Novo Nordisk A/S,**
**Corporate Patents,**
**Novo Allé**
**2880 Bagsvaerd (DK)**

(56) References cited:
EP-A1- 0 692 489          WO-A1-96/40089
WO-A1-97/48413          WO-A1-98/42368
US-A- 5 474 978          US-A- 5 506 203

**Description**

**Field of the invention**

The present invention relates to stable, aqueous insulin formulations suitable for pulmonary delivery with increased convenience for the patient and improved bioavailability of insulin.

**Background of the invention**

Diabetes is a general term for disorders in man having excessive urine excretion as in diabetes mellitus and diabetes insipidus. Diabetes mellitus is a metabolic disorder in which the ability to utilize glucose is more or less completely lost. About 2 % of all people suffer from diabetes.

Since the introduction of insulin in the 1920's, continuous strides have been made to improve the treatment of diabetes mellitus. To help avoid extreme glycaemia levels, diabetic patients often practice multiple injection therapy, whereby insulin is administered with each meal.

In solution, the self-association pattern of insulin is a complex function of protein concentration, metal ions, pH, ionic strength and solvent composition. For the currently used soluble preparations containing U100 insulin, zinc ions, isotonic agent and phenolic preservative, the following equilibria must be considered:

$$6 \text{ In} \leftrightarrow 3 \text{ In}_2$$

$$3 \text{ In}_2 + 2 \text{ Zn}^{2+} \leftrightarrow \text{In}_6 \ (\text{T}_6)$$

$$\text{T}_6 \leftrightarrow \text{T}_3\text{R}_3 \leftrightarrow \text{R}_6$$

The known degradation patterns of insulin include a) fibril formation; b) deamidations at A18, A21 and B3; c) dimerisations via transamidation or Schiff-base formation; d) disulfide exchange reactions.

According to Brange (Stability of Insulin, Kluwer Academic Press,1994), each of these degradation reactions proceed much faster in the monomeric state than in the hexameric state. Therefore, the most efficient means of stabilising insulin preparations is by pushing the above equilibrium as far to the right as possible. In addition to this general effect of mass action, the reactivity of selected residues is further modified depending on their direct involvement in the T → R conformational change. Thus, the reactivity of B3Asn is much lower in the R-state (when the residue resides in an α-helix) than in the T-state.

The interconversion between $\text{T}_6$, $\text{T}_3\text{R}_3$ and $\text{R}_6$ conformations of the two zinc insulin hexamer is modulated by ligand binding to the $\text{T}_3\text{R}_3$ and $\text{R}_6$ forms. Anions such as chloride have affinity for the fourth coordination position in the metal ions of $\text{T}_3\text{R}_3$ and $\text{R}_6$, while preservatives such as phenol binds to hydrophobic pockets located near the surfaces of the $\text{T}_3\text{R}_3$ and $\text{R}_6$ forms (Derewenda, Nature 338, 594, 1989 and, Brzovic, Biochemistry 33, 130557,1994). By the use of $\text{Co}^{2+}$ insulin it has been shown that the combined effect of anion and phenol binding is particularly efficient in stabilising the $\text{R}_6$ state. (Brader, Trends Biochem. Sci. 30, 6636, 1991 and; Bloom, J. Mol. Biol. 245, 324, 1995). Furthermore, for both $\text{Zn}^{2+}$- and $\text{Co}^{2+}$ insulin it has been shown that phenol is much more efficient than m-cresol in inducing R-state in the insulin hexamer (Wollmer, Biol. Chem. Hoppe-Seyler 368, 903, 1987 and, Choi, Biochemistry 32,11638,1993). High affinity phenol derivatives inducing R-state are 7-hydroxy-indol ((Dodson, Phil. Trans. R. Soc. Lond. A 345,153, 1993) resorcinol and 2,6- and 2,7-dihydroxy-naphtalen ((Bloom, J. Mol. Biol. 245, 324, 1995).

The physical denaturation of insulin is known as fibrillation. In the fibrillar state extended peptide chains are laying parallel or anti parallel and hydrogen bonded to each other, so-called β-structure or β-pleated sheets. Fibrils represent usually the lowest state of energy of the protein, and only harsh conditions such as strong base may enable a regeneration from this state to the native state of correctly folded protein. Factors that promote the rate of formation of fibrils are increasing the temperature, increasing the surface area between the liquid and the air phase and, for zinc-free insulin, increasing the concentration. For hexameric zinc-insulin the rate of fibril formation decreases with increasing concentration. The formation of fibrils is believed to proceed via monomerization of insulin. Fibrils of insulin have the appearance of gels or precipitates.

Insulin derivatives having truncations in the C-terminal of the B-chain, e.g. despentapeptide (B26-B30) insulin and desoctapeptide (B23-B30) insulin are more prone to form fibrils than human insulin. Insulin analogues which dissociate readily from the hexameric unit to the monomeric form, e.g. the AspB28 human insulin and the LysB28-ProB29 human insulin, are likewise more prone to form fibrils than human insulin.

The native state of insulin is stabilised by bringing about the conditions that stabilises the hexameric unit, i.e. the presence of zinc ions (2-4 zinc/hexamer), phenol (0.1-0.5% w/v) and sodium chloride (5-150 mM).

Addition of agents that reduce the surface tension at the air-liquid interface further reduces the propensity to fibril formation. Thus, polyethylene glycol, polypropylene glycol and copolymers hereof with an average molecular weights of about 1800 have found use as stabilisers in concentrated insulin solutions for infusion pumps (Grau, 1982. In: Neue Insuline (Eds. Petersen, Schlüter & Kerp), Freiburger Graphische Betriebe, pp. 411-419 and Thurow, 1981: patent DE2952119A1). For a comprehensive review on the physical stability of insulin see Brange 1994, Stability of Insulin, Kluwer Academic Publisher, pp. 18-23.

Most of the chemical degradation of insulin in preparations is due to reactions involving the carboxamide function of the asparagine residues, in particular residues B3 and A21. Hydrolysis of the amide groups leads to desamido derivatives, and transamidation involving an amino group from another molecule leads to covalently linked dimers and, after similar consecutive reactions, to trimers and higher polymers.

In acid solution AsnA21 is the most reactive, leading to AspA21 insulin (Sundby, J. Biol. Chem. 237, 3406, 1962). In crude insulin of bovine and porcine origin, obtained by acid ethanol extraction, the most abundant dimers isolated were AspA21 -GlyA1 and AspA21 - PheB1 linked (Helbig 1976, Insulindimere aus der B-Komponente von Insulinpräparationen, Thesis at the Rheinisch-Westfälischen Technischen Hochschule, Aachen).

In neutral solution, which is the preferred embodiment of insulin preparations for injection therapy, AsnB3 is the most susceptible residue. Degradation products include AspB3 insulin, AspB3 -GlnB4 isopeptide insulin, and dimers and higher polymers where AspB3 provides the carbonyl moiety of a peptide bond with an amino group of another molecule.

For a comprehensive review on the chemical stability of insulin see Brange 1994, Stability of Insulin, Kluwer Academic Publisher, pp. 23-36. As for the physical stability conditions that stabilises the hexameric unit, i.e. the presence of zinc ions (2-4 zinc/hexamer), phenol (0.1-0.5% w/v) and sodium chloride (5-150 mM), decrease the rate of formation of degradation products during storage at neutral pH.

A different type of polymerisation reaction is observed when the conditions that stabilises the hexameric unit is neglected. Thus, in the absence of zinc, phenol and sodium chloride, and using a temperature of 50°C, disulfide-linked dimers and high molecular weight polymers are the prevailing products formed. The mechanism of formation is a disulfide interchange reaction, resulting from β-elimination of the disulfides (Brems, Protein Engineering 5, 519, 1992).

Solubility of insulin is a function of pH, metal ion concentration, ion strength, phenolic substances, solvent composition (polyols, ethanol and other solvents), purity, and species (bovine, porcine, human, other analogues). For a review see Brange: Galenics of Insulin, Springer-Verlag 1987, p.18 and 46.

The solubility of insulin is low at pH values near its isoelectric pH, i.e. in the pH range 4.0 - 7.0. Highly concentrated solutions of porcine insulin (5000 U/ml $\sim$ 30 mM) have been brought about at acid pH (Galloway, Diabetes Care 4, 366, 1981), but the insulin in the formulation is highly instable due to deamidation at AsnA21. At neutral pH highly concentrated solutions of zinc free insulin can be made, but these are unstable due to a high rate of polymerisation and deamidation at AsnB3. Porcine zinc insulin solutions at neutral pH comprising phenol have been reported physical stable at concentrations of 1000 U/ml at elevated temperature, but become supersaturated when the temperature is lowered to 4 °C. (Brange and Havelund in Artificial Systems for Insulin Delivery, Brunetti et al. eds, Raven Press 1983).

In order to reduce the inconvenience of insulin injections much attention has been given to alternative routes of administration (for an overview see Brange and Langkjaer in Protein Delivery: Physical Systems, Sanders and Hendren, eds., Plenum Press 1997). Pulmonary delivery seems to be the most promising of these (Service, Science 277,1199.1997). Insulin can be given aerolised in the form of dry powder or as nebulised droplets from an insulin solution. The efficacy might be enhanced by coached breathing (Gonda, US Patent 5,743,250) and addition of an absorption enhancer (Baekstroem, US Patent 5,747,445) or protease inhibitors (Okumura, Int. J. Pharm. 88, 63, 1992). The bioavailability of a nebulised concentrated insulin solution (500 U/ml) was shown to be 20-25 % as compared to a subcutaneous injection (Elliot, Aust. Paediatr. J. 23, 293, 1987). By using 30-50 µl insulin solution per puff the insulin solution need to be 5-20 times more concentrated than the usual concentration of 0.6 mM. By using a single dose container, e.g. a blister pack (Gonda, US Patent 5,743,250), the demand for a preservative is abolished. Most insulin formulations are preserved by the toxic, mucose irritating and unpleasant odorous phenol and m-cresol. However, omitting phenols will cause stability problems. In addition to the bacteriostatic efficacy, the phenols act as physicochemical stabilisers of insulin in combination with zinc ions. So, it is preferred that formulations of insulin for inhalation are made with a minimum concentration of phenol or that phenol has been replaced by more acceptable substitutes.

**Description of the invention**

Definitions

By "analogue of human insulin" (and similar expressions) as used herein is meant human insulin in which one or more amino acids have been deleted and/or replaced by other amino acids, including non-codeable amino acids, or human insulin comprising additional amino acids, i.e. more than 51 amino acids.

By "derivative of human insulin" (and similar expressions) as used herein is meant human insulin or an analogue thereof in which at least one organic substituent is bound to one or more of the amino acids.

By "phenols" or "phenolic molecules" as used herein is meant phenol or derivatives thereof such as m-cresol or chloro-cresol.

By "menthol" is meant (-)-menthol and derivatives thereof as well as racemic menthol.

Brief description of the invention

It is an object of the present invention to provide an insulin formulation for pulmonary delivery which has an increased convenience for the patient without deteriorating its physical and chemical stability.

It is also an object of the invention to provide a pulmonary insulin formulation with an improved bioavailability of insulin. Unexpectedly, these objects have been accomplished by providing an insulin formulation in which the amount of toxic and mucose irritating phenols has been minimized and in which menthol has been added.

Accordingly, the present invention relates to an aqueous insulin formulation comprising: human insulin or an analogue or a derivative thereof, 2 to 5 $Zn^{2+}$ ions per six molecules of insulin, 3 to 18 phenolic molecules per six molecules of insulin, and menthol.

The characteristic smell of menthol masks the presence of unpleasant phenols in the formulation and, surprisingly, the chemical and physical stability is not adversely affected by menthol.

Furthermore, the presence of menthol alleviates the sensation of respiratory discomfort associated with the act of inhaling, improve inspiratory and exspiratory volume, and mediates an antitussive effect.

Moreover, an increase in the bioavailability of insulin can be observed in comparison with formulations without menthol.

As an alternative to menthol, eucalypthol and related substances may be used according to the present invention. Furthermore, the smell might be adjusted by a mixture of these compounds.

**Preferred embodiments**

The insulin formulation according to the present invention preferably comprises 0.5 to 4 mM of menthol.

The amount of phenolic molecules in the insulin formulation preferably corresponds to 4 to 9 phenolic molecules per six molecules of insulin, more preferably to about 6 phenolic molecules per six molecules of insulin.

The phenolic molecules are preferably selected from the group consisting of phenol, m-cresol, chloro-cresol, thymol, or any mixture thereof.

The insulin formulation preferably contains 0.3 to 20 mM, more preferably 0.6 to 15 mM, still more preferably 3 to 12 mM of human insulin or an analogue or a derivative thereof.

The stability of the insulin formulation is further improved when the concentration of chloride is kept below 50 mM, preferably below 30 mM, and more preferably in the range of 5 to 20 mM.

A remarkable stability of the insulin formulation is obtained when it comprises less than 10 mM of any anions other than chloride and acetate.

In a particular embodiment the insulin may comprise a low amount of phosphate buffer, preferably up to 5 mM of phosphate.

Insulin formulations of the invention comprising 2 to 4 $Zn^{2+}$ ions, preferably 2.2 to 3.2 $Zn^{2+}$ ions per six molecules of insulin, are very stable.

Insulin formulations of the invention comprising 3 to 5 $Zn^{2+}$ ions, preferably 3.5 to 5 $Zn^{2+}$ ions per six molecules of insulin, are also advantageous.

Surprisingly, it is possible to add a relatively high concentrations of zwitterions such as glycylglycine and glycine to the insulin formulation of the invention without decreasing the solubility of insulin. Glycylglycine act as buffer at neutral pH and furthermore increase the dissolution rate of zinc insulin at neutral to basic pH due to a moderately zinc chelating effect. Also, glycylglycine may act as a scavenger for amine reactions during the storage time. Thus, in a preferred embodiment the insulin formulation of the invention further comprises 5 to 150 mM of a zwitterionic amine, preferably glycylglycine or glycine.

In a preferred embodiment the insulin formulation of the invention further comprises 5 to 50 mM of trishydroxymethylaminomethan, which acts as a buffer at neutral pH and as a scavenger for amine reactive compounds.

In another preferred embodiment the insulin formulation of the invention comprises sodium ions as cations. The sodium ion has a low salting out effect.

In another preferred embodiment the insulin formulation of the invention comprises potassium or a mixture of potassium and sodium ions as cations. Potassium ions in a concentration higher than the plasma concentration of 4-5 mM increase the transport of insulin through the lungs.

In another preferred embodiment potassium ion in a concentration more than 4-5 mM is used in combination with a mild bronchodilator such as menthol.

In another preferred embodiment the insulin formulation of the invention comprises between 0.001 % by weight and 1 % by weight of a non-ionic surfactant, preferably tween 20 or Polox 188. A nonionic detergent can be added to stabilise insulin against fibrillation during storage and nebulisation.

In another preferred embodiment the insulin formulation of the invention comprises 1 mM to 10 mM of an anionic surfactant, preferably sodium taurocholate, in order to further increase the bioavailabilty of insulin.

In a preferred embodiment the insulin used is human insulin.

In another preferred embodiment the insulin used is an analogue of human insulin wherein position B28 is Asp, Lys, Leu, Val or Ala and position B29 is Lys or Pro; or des(B28-B30), des(B27) or des(B30) human insulin.

The preferred analogues of human insulin are those in which position B28 is Asp or Lys, and position B29 is Lys or Pro, preferably $Asp^{B28}$ human insulin or $Lys^{B28}Pro^{B29}$ human insulin.

In another preferred embodiment the insulin is selected from the group of soluble long-acting insulin derivatives such as derivatives of human insulin having one or more lipophilic substituents, preferably acylated insulins.

The insulin derivative according to this embodiment is preferably selected from the group consisting of B29-N$^\varepsilon$-myristoyl-des(B30) human insulin, B29-N$^\varepsilon$-palmitoyl-des(B30) human insulin, B29-N$^\varepsilon$-myristoyl human insulin, B29-N$^\varepsilon$-palmitoyl human insulin, B28-N$^\varepsilon$-myristoyl $Lys^{B28}$ $Pro^{B29}$ human insulin, B28-N$^\varepsilon$-palmitoyl $Lys^{B28}$ $Pro^{B29}$ human insulin, B30-N$^\varepsilon$-myristoyl-$Th^{B29}Lys^{B30}$ human insulin, B30-N$^\varepsilon$-palmitoyl-$Thr^{B29}Lys^{B30}$ human insulin, B29-N$^\varepsilon$-(N-palmitoyl-$\gamma$-glutamyl)-des(B30) human insulin, B29-N$^\varepsilon$-(N-lithocholyl-$\gamma$-glutamyl)-des(B30) human insulin, B29-N$^\varepsilon$-($\omega$-carboxyheptadecanoyl)-des(B30) human insulin and B29-N$^\varepsilon$-($\omega$-carboxyheptadecanoyl) human insulin.

The most preferred insulin derivative is B29-N$^\varepsilon$-myristoyl-des(B30) human insulin or B29-N$^\varepsilon$-(N-lithocholyl-$\gamma$-glutamyl)-des(B30) human insulin.

The above mentioned soluble long acting insulin derivatives bind albumin and have been designed to provide a constant basal supply of insulin (Markussen, Diabetologia 39, 281, 1996). Subcutaneous administration once or twice daily secure the required basal delivery of insulin, whereas several daily inhalations are recommended using pulmonary administration, preferably in connection with meals.

The insulin derivatives have a protracted onset of action and may thus compensate the very rapid increase in plasma insulin normally associated with pulmonary administration.

By careful selection of the type of insulin, the present invention enables adjustment of the timing, and in order to obtain the desired insulin profile.

In a particular embodiment of the present invention, the insulin formulation comprises an insulin analogue or human insulin as well as an insulin derivative.

The insulin formulations of present inventions preferably has a pH value in the range of 7 to 8.5, more preferably 7.4 to 7.9 to ensure optimum stability.

This invention is further illustrated by the following examples which, however, are not to be construed as limiting. The term "Equivalent" is used as stoichiometric amount relative to insulin.

EXAMPLE 1

Human zinc insulin was dispersed in water (1:10 (w/w)) on icebath. After gentle stirring glycylglycine (7/15 equivalent) and sodium hydroxide (3.1 equivalent) were added and the mixture stirred slowly at 5 °C until the insulin was dissolved. 0.1 equivalent of zinc chloride and detergent was added. The pH was adjusted to 7.5 by 0.8 equivalent of hydrochloric acid and the volume adjusted before adding phenol (0, 0.67, 1, and 1.33 equivalent per insulin) or chlorcresol (1 equivalent) or cresol (1 equivalent), menthol (0, 1 and 2 mM from a stock solution of 1 M in ethanol), and water. Finally the 15 mM preparation was diluted with medium containing sodium chloride, glycylglycine, detergent, and menthol to obtain 12, 9, 6, and 3 mM of human insulin. (Table 1).

The odour of the solutions was evaluated by smelling directly over 1 ml of final solution of 9 mM of insulin. The smell of phenol was very weak at 0.67 equivalents of phenol per insulin and increasing to moderate at 1.33 equivalents. Adding 1 mM of menthol masked the smell of phenol at the 3 levels, and at 2 mM of menthol the mentholic odour was pronounced. 1 mM of menthol also masked the smell of 1 equivalent of chlorcresol per insulin. The smell of cresol was distinct at 1 mM of menthol and almost neutralised at 2 mM menthol.

The chemical stability of insulin was measured as the rate of covalent polymerisation. The polymerisation of insulin was not adversely affected by menthol.

The results are presented in the following Table 1.

Table 1

| Excipient | Physical stability of solution at 5 °C | Chemical stability at 37 °C | |
|---|---|---|---|
| 0.5 Zn$^{2+}$/insulin, NaCl 15 mM glycylglycine 7 mM, tween 20 0.01%, pH 7.5 and: | Max. concentration without precipitation for 1 week. Test solutions were 3, 6, 9, 12, 15 mM insulin. | % polymer / week 3 and 15 mM insulin | |
| reference without phenols | 15 | 0.71 | 1.02 |
| 0.67 eqv phenol | 15 | 0.37 | 0.48 |
| 0.67 eqv phenol, 1 mM menthol | 15 | 0.42 | 0.47 |
| 0.67 eqv phenol, 2 mM menthol | 15 | 0.42 | 0.48 |
| 1 eqv phenol | 15 | 0.32 | 0.29 |
| 1 eqv phenol, 1 mM menthol | 15 | 0.32 | 0.30 |
| 1 eqv phenol, 2 mM menthol | 15 | 0.26 | 0.23 |
| 1.33 eqv phenol | 15 | 0.33 | 0.27 |
| 1.33 eqv phenol, 1 mM menthol | 15 | 0.27 | 0.21 |
| 1.33 eqv phenol, 2 mM menthol | 15 | 0.30 | 0.21 |
| 1 eqv cresol, 1mM menthol | 15 | 0.45 | 0.38 |
| 1 eqv chloro-cresol, 1 mM menthol | 15 | 0.25 | 0.25 |
| 1 eqv phenol, 1 mM menthol, without tween 20 | 15 | 0.33 | 0.33 |
| 1 eqv phenol, 1 mM menthol, tween 20 replaced by Polox188 | 15 | 0.36 | 0.35 |
| phenol and cresol 16 mM | 15 | 0.22 | 0.19 |
| phenol and cresol 16 mM, menthol 1 mM | 15 | 0.24 | 0.18 |
| phenol and cresol 16 mM, menthol 2 mM | 15 | 0.24 | 0.19 |

**Claims**

1. An aqueous insulin formulation comprising: human insulin or an analogue or a derivative thereof, 2 to 5 Zn$^{2+}$ ions per six molecules of insulin, 3 to 18 phenolic molecules per six molecules of insulin, and menthol.

2. An insulin formulation according to claim 1 comprising 0.5 to 4 mM of menthol.

3. An insulin formulation according to claim 1 or 2 comprising 4 to 9 phenolic molecules per six molecules of insulin, preferably about 6 phenolic molecules per six molecules of insulin.

4. An insulin preparation according to any of the preceding claims, wherein the phenolic molecules are selected from the group consisting of phenol, m-cresol, chloro-cresol, thymol, or any mixture thereof.

5. An insulin formulation according to any of the preceding claims comprising 0.3 to 20 mM, preferably 0.6 to 15 mM, more preferably 3 to 12 mM of human insulin or an analogue or a derivative thereof.

6. An insulin formulation according to any of the preceding claims comprising less than 50 mM, preferably less than 30 mM of chloride, more preferably 5 to 20 mM of chloride.

7. An insulin formulation according to any of the preceding claims comprising less than 10 mM of any anions other than chloride and acetate.

8. An insulin formulation according to any of the preceding claims comprising up to 5 mM of phosphate.

9. An insulin formulation according to any of the preceding claims comprising 2 to 4 $Zn^{2+}$ ions, preferably 2.2 to 3.2 $Zn^{2+}$ ions per six molecules of insulin.

10. An insulin formulation according to any of the preceding claims, further comprising 5 to 150 mM of a zwitterionic amine, preferably glycylglycine or glycine.

11. An insulin formulation according to any of the preceding claims, further comprising 5 to 50 mM of trishydroxymethylaminomethan.

12. An insulin formulation according to any of the preceding claims comprising sodium ions, potassium ions, or a mixture thereof as cations.

13. An insulin formulation according to any of the preceding claims, further comprising between 0.001 % by weight and 1 % by weight of a surfactant, preferably tween 20 or Polox 188.

14. An insulin formulation according to any of the preceding claims comprising human insulin.

15. An insulin preparation according to any of the claims 1 to 13, comprising an analogue of human insulin wherein position B28 is Asp, Lys, Leu, Val or Ala and position B29 is Lys or Pro; or des(B28-B30), des(B27) or des(B30) human insulin.

16. An insulin preparation according to claim 15, comprising an analogue of human insulin wherein position B28 is Asp or Lys, and position B29 is Lys or Pro, preferably $Asp^{B28}$ human insulin or $Lys^{B28}Pro^{B29}$ human insulin.

17. An insulin preparation according to any one of the claims 1 to 13, comprising a derivative of human insulin having one or more lipophilic substituents, preferably an acylated insulin.

18. An insulin preparation according to claim 17, wherein the insulin derivative is selected from the group consisting of B29-$N^{\varepsilon}$-myristoyl-des(B30) human insulin, B29-$N^{\varepsilon}$-palmitoyl-des(B30) human insulin, B29-$N^{\varepsilon}$-myristoyl human insulin, B29-$N^{\varepsilon}$-palmitoyl human insulin, B28-$N^{\varepsilon}$-myristoyl $Lys^{B28}$ $Pro^{B29}$ human insulin, B28-$N^{\varepsilon}$-palmitoyl $Lys^{B28}$ $Pro^{B29}$ human insulin, B30-$N^{\varepsilon}$-myristoyl-$Thr^{B29}Lys^{B30}$ human insulin, B30-$N^{\varepsilon}$-palmitoyl-$Thr^{B29}Lys^{B30}$ human insulin, B29-$N^{\varepsilon}$-(N-palmitoyl-$\gamma$-glutamyl)-des(B30) human insulin, B29-$N^{\varepsilon}$-(N-lithocholyl-$\gamma$-glutamyl)-des(B30) human insulin, B29-$N^{\varepsilon}$-($\omega$-carboxyheptadecanoyl)-des(B30) human insulin and B29-$N^{\varepsilon}$-($\omega$-carboxyheptadecanoyl) human insulin.

19. An insulin preparation according to claim 18, wherein the insulin derivative is B29-$N^{\varepsilon}$-myristoyl-des(B30) human insulin or B29-$N^{\varepsilon}$-(N-lithocholyl-$\gamma$-glutamyl)-des(B30) human insulin.

20. An insulin preparation according to any one of the preceding claims, comprising an insulin analogue or human insulin as well as an insulin derivative.

21. An insulin preparation according to any one of the preceding claims, having a pH value in the range of 7 to 8.5, preferably 7.4 to 7.9.

22. Use of an insulin formulation according to any of the preceding claims for the manufacture of a medicament for the treatment of type I or type II diabetes.

23. Use according to claim 22, where the insulin is to be administered or connection with meals.

**Patentansprüche**

1. Wässrige Insulinformulierung umfassend: Humaninsulin oder ein Analogon oder ein Derivat davon, 2 bis 5 $Zn^{2+}$

Ionen pro sechs Moleküle Insulin, 3 bis 18 phenolische Moleküle pro sechs Moleküle Insulin und Menthol.

2.  Insulinformulierung nach Anspruch 1 umfassend 0,5 bis 4 mM Menthol.

3.  Insulinformulierung nach Anspruch 1 oder 2 umfassend 4 bis 6 phenolische Moleküle pro sechs Moleküle Insulin, vorzugsweise etwa 6 phenolische Moleküle pro sechs Moleküle Insulin.

4.  Insulinzubereitung nach jedem der vorangehenden Ansprüche, wobei die phenolischen Moleküle ausgewählt sind aus der Gruppe bestehend aus Phenol, m-Kresol, Chlorkresol, Thymol oder jedem Gemisch davon.

5.  Insulinformulierung nach jedem der vorangehenden Ansprüche umfassend 0,3 bis 20 mM, vorzugsweise 0,6 bis 15 mM, mehr bevorzugt 3 bis 12 mM Humaninsulin oder ein Analogon oder ein Derivat davon.

6.  Insulinformulierung nach jedem der vorangehenden Ansprüche umfassend weniger als 50 mM, vorzugsweise weniger als 30 mM Chlorid, noch mehr bevorzugt 5 bis 20 mM Chlorid.

7.  Insulinformulierung nach jedem der vorangehenden Ansprüche umfassend weniger als 10 mM irgendwelcher Anionen außer Chlorid und Acetat.

8.  Insulinformulierung nach jedem der vorangehenden Ansprüche umfassend bis zu 5 mM Phosphat

9.  Insulinformulierung nach jedem der vorangehenden Ansprüche umfassend 2 bis 4 $Zn^{2+}$-Ionen, vorzugsweise 2,2 bis 3,2 $Zn^{2+}$-Ionen pro sechs Moleküle Insulin.

10. Insulinformulierung nach jedem der vorangehenden Ansprüche weiterhin umfassend 5 bis 150 mM eines zwitterionischen Amins, vorzugsweise Glycylglycin oder Glycin.

11. Insulinformulierung nach jedem der vorangehenden Ansprüche weiterhin umfassend 5 bis 50 mM Trihydroxymethylaminomethan.

12. Insulinformulierung nach jedem der vorangehenden Ansprüche umfassend Natriumionen, Kaliumionen oder ein Gemisch davon als Kationen.

13. Insulinformulierung. nach jedem der vorangehenden Ansprüche weiterhin umfassend zwischen 0,001% nach Gewicht und 1% nach Gewicht einer oberflächenaktive Substanz, vorzugsweise Tween 20 oder Polox 188.

14. Insulinformulierung nach jedem der vorangehenden Ansprüche umfassend Humaninsulin.

15. Insulinzubereitung nach jedem der Ansprüche 1 bis 13 umfassend ein Analogon des Humaninsulins, wobei die Position B28 Asp, Lys, Leu, Val oder Ala ist und die Position B29 Lys oder Pro ist oder Des(B28-B30)-, Des(B27)- oder Des(B30)-humaninsulin.

16. Insulinzubereitung nach Anspruch 15 umfassend ein Analogon des Humaninsulins, wobei die Position B28 Asp oder Lys und die Position B29 Lys oder Pro ist, vorzugsweise $Asp^{B28}$-Humaninsulin oder $Lys^{B28}Pro^{B29}$-Humaninsulin.

17. Insulinzubereitung nach jedem der Ansprüche 1 bis 13 umfassend ein Derivat des Humaninsulins, welches einen oder mehrere lipophile Substituenten hat, vorzugsweise ein acyliertes Insulin.

18. Insulinzubereitung nach Anspruch 17, wobei das Insulinderivat ausgewählt ist aus der Gruppe bestehend aus B29-$N^{\epsilon}$-Myristyl-des(B30)-humaninsulin, B29-$N^{\epsilon}$-Palmityl-des(B30)-humaninsulin, B29-$N^{\epsilon}$-Myristylhumaninsulin, B29-$N^{\epsilon}$-Palmitylhumaninsulin, B28-$N^{\epsilon}$-Myristyl-$Lys^{B28}Pro^{B29}$-humaninsulin, B28-$N^{\epsilon}$-Palmityl-$Lys^{B28}Pro^{B29}$-humaninsulin, B30-$N^{\epsilon}$-Myristyl-$Thr^{B29}Lys^{B30}$-humaninsulin, B30-$N^{\epsilon}$-Palmityl-$Thr^{B29}Lys^{B30}$-humaninsulin, B29-$N^{\epsilon}$-(N-Palmityl-$\gamma$-glutamyl)-des(B30)-humaninsulin, B29-$N^{\epsilon}$-(N-Lithocholyl-$\gamma$-glutamyl)-des(B30)-humaninsulin, B29-$N^{\epsilon}$-($\omega$-Carboxyheptadecanyl)-des(B30)-human-insulin und B29-$N^{\epsilon}$-($\omega$-Carboxyheptadecanyl)-humaninsulin.

19. Insulinzubereitung nach Anspruch 18, wobei das Insulinderivat B29-$N^{\epsilon}$-Myristyl-des(B30)-humaninsulin oder B29-$N^{\epsilon}$-(N-Lithocholyl-$\gamma$-glutamyl)-des(B30)-humaninsulin ist.

**20.** Insulinzubereitung nach jedem der vorangehenden Ansprüche umfassend ein Insulinanalogon oder Humaninsulin sowie ein Insulinderivat.

**21.** Insulinzubereitung nach jedem der vorangehenden Ansprüche, die einen pH-Wert im Bereich von 7 bis 8,5, vorzugsweise von 7,4 bis 7,9 hat.

**22.** Verwendung einer Insulinformulierung nach jedem der vorangehenden Ansprüche zur Herstellung eines Medikaments zur Behandlung von Typ I oder Typ II Diabetes.

**23.** Verwendung nach Anspruch 22, wobei das Insulin im Zusammenhang mit einer Mahlzeit verabreicht werden soll.


**Revendications**

**1.** Formulation aqueuse d'insuline comprenant : de l'insuline humaine ou un analogue ou un dérivé de celle-ci, 2 à 5 ions $Zn^{2+}$ pour six molécules d'insuline, 3 à 18 molécules phénoliques pour six molécules d'insuline, et du menthol.

**2.** Formulation d'insuline selon la revendication 1, comprenant du menthol à une concentration de 0,5 à 4 mM.

**3.** Formulation d'insuline selon la revendication 1 ou 2, comprenant 4 à 9 molécules phénoliques pour six molécules d'insuline, de préférence environ 6 molécules phénoliques pour six molécules d'insuline.

**4.** Préparation d'insuline selon l'une quelconque des revendications précédentes, dans laquelle les molécules phénoliques sont choisies dans l'ensemble constitué par le phénol, le m-crésol, le chloro-crésol, le thymol, ou l'un quelconque de leurs mélanges.

**5.** Formulation d'insuline selon l'une quelconque des revendications précédentes, comprenant de l'insuline humaine ou un analogue ou un dérivé de celle-ci à une concentration de 0,3 à 20 mM, de préférence de 0,6 à 15 mM, mieux encore de 3 à 12 mM.

**6.** Formulation d'insuline selon l'une quelconque des revendications précédentes, comprenant une concentration de chlorure inférieure à 50 mM, de préférence inférieure à 30 mM, mieux encore de 5 à 20 mM.

**7.** Formulation d'insuline selon l'une quelconque des revendications précédentes, comprenant une concentration de quelconques anions autres que les chlorures et acétates inférieure à 10 mM.

**8.** Formulation d'insuline selon l'une quelconque des revendications précédentes, comprenant une concentration de phosphate allant jusqu'à 5 mM.

**9.** Formulation d'insuline selon l'une quelconque des revendications précédentes, comprenant 2 à 4 ions $Zn^{2+}$, de préférence 2,2 à 3,2 ions $Zn^{2+}$ pour six molécules d'insuline.

**10.** Formulation d'insuline selon l'une quelconque des revendications précédentes, comprenant en outre une amine zwittérionique, de préférence la glycylglycine ou la glycine, à une concentration de 5 à 150 mM.

**11.** Formulation d'insuline selon l'une quelconque des revendications précédentes, comprenant en outre du trishydroxyméthylaminométhane à une concentration de 5 à 50 mM.

**12.** Formulation d'insuline selon l'une quelconque des revendications précédentes, comprenant des ions sodium, des ions potassium, ou un mélange de ceux-ci à titre de cations.

**13.** Formulation d'insuline selon l'une quelconque des revendications précédentes, comprenant en outre entre 0,001 et 1 % en poids d'un tensioactif, de préférence le Tween 20 ou le Polox 188.

**14.** Formulation d'insuline selon l'une quelconque des revendications précédentes, comprenant de l'insuline humaine.

**15.** Préparation d'insuline selon l'une quelconque des revendications 1 à 13, comprenant un analogue d'insuline humaine dans lequel la position B28 est Asp, Lys, Leu, Val ou Ala et la position B29 est Lys ou Pro ; ou l'insuline humaine des(B28-B30), des(B27) ou des(B30).

16. Préparation d'insuline selon la revendication 15, comprenant un analogue d'insuline humaine dans lequel la position B28 est Asp ou Lys, et la position B29 est Lys ou Pro, de préférence l'insuline humaine Asp$^{B28}$ ou l'insuline humaine Lys$^{B28}$Pro$^{B29}$.

17. Préparation d'insuline selon l'une quelconque des revendications 1 à 13, comprenant un dérivé d'insuline humaine ayant un ou plusieurs substituants lipophiles, de préférence une insuline acylée.

18. Préparation d'insuline selon la revendication 17, dans laquelle le dérivé d'insuline est choisi dans l'ensemble constitué par l'insuline humaine B29-N$^\varepsilon$-myristoyl-des(B30), l'insuline humaine B29-N$^\varepsilon$-palmitoyldes(B30), l'insuline humaine B29-N$^\varepsilon$-myristoyle, l'insuline humaine B29-N$^\varepsilon$-palmitoyle, l'insuline humaine B29-N$^\varepsilon$-myristoyl-Lys$^{B28}$Pro$^{B29}$, l'insuline humaine B28-N$^\varepsilon$-palmitoyl-Lys$^{B28}$Pro$^{B29}$, l'insuline humaine B30-N$^\varepsilon$-myristoyl-Thr$^{B28}$Lys$^{B30}$, l'insuline humaine B30-N$^\varepsilon$-palmitoyl-Thr$^{B29}$Lys$^{B30}$, l'insuline humaine B29-N$^\varepsilon$-(N-palmitoyl-$\gamma$-glutamyl)-des(B30), l'insuline humaine B29-N$^\varepsilon$-(N-lithocholyl-$\gamma$-glutamyl)-des(B30), l'insuline humaine B29-N$^\varepsilon$-($\omega$-carboxyheptadécanoyl)-des(B30) et l'insuline humaine B29-N$^\varepsilon$-($\omega$-carboxyheptadécanoyle) .

19. Préparation d'insuline selon la revendication 18, dans laquelle le dérivé d'insuline est l'insuline humaine B29-N$^\varepsilon$-myristoyl-des(B30) ou l'insuline humaine B29-N$^\varepsilon$-(N-lithocholyl-$\gamma$-glutamyl)-des(B30).

20. Préparation d'insuline selon l'une quelconque des revendications précédentes, comprenant un analogue d'insuline ou de l'insuline humaine ainsi qu'un dérivé d'insuline.

21. Préparation d'insuline selon l'une quelconque des revendications précédentes, ayant une valeur de pH située dans la plage allant de 7 à 8,5, de préférence de 7,4 à 7,9.

22. Utilisation d'une formulation d'insuline selon l'une quelconque des revendications précédentes, pour la fabrication d'un médicament destiné au traitement du diabète de type I ou de type II.

23. Utilisation selon la revendication 22, où l'insuline doit être administrée en relation avec les repas.